# EUROPEAN PATENT SPECIFICATION

(11) **EP 1 976 520 B1**
(45) Date of publication and mention of the grant of the patent: **27.04.2011**
(21) Application number: 06847938.5
(22) Date of filing: 22.12.2006
(51) Int. Cl.: A61K 31/4402, A61K 31/77, A61K 45/06, A61P 1/00

(54) **KIT COMPRISING AN OSMOTIC LAXATIVE AND A STIMULANT LAXATIVE FOR PREPARING THE COLON FOR VIRTUAL COLONOSCOPY**
KITS MIT EINEM OSMOTISCHEN LAXANS UND EINEM STIMULIERENDEN LAXANS ZUR VORBEREITUNG DES KOLON AUF EINE VIRTUELLE KOLONOSKOPIE
KIT COMPRENANT UN LAXATIF OSMOTIQUE ET UN LAXATIF STIMULANT POUR LA PREPARATION DU COLON POUR UNE COLOSCOPIE VIRTUELLE

(30) Priority: 29.12.2005 US 754721 P; 09.01.2006 US 757829 P
(43) Date of publication of application: 08.10.2008
(73) Proprietor: Braintree Laboratories, Inc., Braintree MA 02185-0929 (US)
(72) Inventor: PELHAM, Russell, W., Duxbury, MA 02332 (US)
(74) Representative: Grund, Martin
(86) International application number: PCT/US2006/048838
(87) International publication number: WO 2007/079000

(56) References cited:
- WO-A-2005/061008
- PICKHARDT PERRY J ET AL: "Computed tomographic virtual colonoscopy to screen for colorectal neoplasia in asymptomatic adults." NEW ENGLAND JOURNAL OF MEDICINE, vol. 349, no. 23, 4 December 2003 (2003-12-04), pages 2191-2200, XP002452076 ISSN: 0028-4793
- BRADY C E ET AL: "EFFECT OF BISACODYL ON GUT LAVAGE CLEANSING FOR COLONOSCOPY" ANNALS OF CLINICAL RESEARCH, HELSINKI, FI, vol. 19, no. 1, 1987, pages 34-38, XP009024250 ISSN: 0003-4762
- CHEN C-C ET AL: "3-D ENDOSCOPIC ULTRASONOGRAPHY AND BETTER COLONOSCOPIC PREPARATION MAGNESIUM CITRATE-BISACODYL REGIMEN PROVES BETTER THAN CASTOR OIL FOR COLONOSCOPIC PREPARATION" JOURNAL OF GASTROENTEROLOGY AND HEPATOLOGY, MELBOURNE, AU, vol. 14, no. 12, 1999, pages 1219-1222, XP001121339 ISSN: 0815-9319

## Description

### Field of the Invention

This invention is in the field of medicine. More specifically this invention relates to gastroenterology, and particularly, a method of cleansing a patient's bowels prior to a virtual colonoscopy procedure.

### Background Information

Although years of clinical research have been expended to make early detection of colorectal cancer (CRC) a high clinical priority, the disease remains the second leading cause of cancer-related death in the United States. In 2003, an estimated 60,000 deaths were attributed to CRC which accounted for the third highest number of new cancer cases that year, only lagging prostate and respiratory cancers for men and breast and respiratory cancers in women. Investigators estimate there will be 147,000 new cases of CRC in 2005 alone. Lifetime risk for developing CRC is therefore estimated to be one case per 18 people in the United States.

Despite the success of screening procedures, which can reduce the rate of death by detecting early cancer or premalignant polyps, about 1,000,000 high-risk people in the United States alone have not been properly screened for colorectal polyps. Reasons given for this low rate of screening include the reluctance of physicians, patients and health care providers to encourage, receive, or pay for these procedures. This has been recognized most recently at the federal level on July 10, 2002 when a U.S. Senate committee voted to require all private health insurance plans in the United States to provide coverage for colonoscopies and other tests to detect colon cancer in people who are 50 or older or have a high risk of developing the disease.

The most common method for screening for colorectal polyps is conventional colonoscopy. Conventional colonoscopy requires sedation of the patient, followed by examination of the colon through the insertion of a five-foot long flexible, lighted tube through the rectum. The procedure lasts about one to two hours. Afterwards, the patient must recover from the sedation and often is incapacitated for the remainder of the day. Due to the invasive nature of conventional colonoscopy and the need for sedation during the procedure, many patients avoid colonoscopies. Other methods such as sigmoidoscopy and digital rectal examination evaluate only a part of the colon and thus are not as thorough as a colonoscopy procedure.

Virtual colonoscopy (VC) is a new method of screening the colon for precancerous polyps. Using a computed tomography (CT) scanner and new computer methods of rendering or reconstructing the images, the colon can be evaluated without a colonoscope and without sedation. After cleansing, the colon is distended with air or carbon dioxide and the CT scan is performed in seconds. The patient is scanned both on his stomach as well as on his back to allow excess fluid and stool to fall away from the dependent portion of the colon.

Current colonoscopy cleansing procedures have included reduced food intake combined with laxatives, enemas, suppositories, bowel evacuants, or orthograde colonic lavage. Orthograde lavage with polyethylene glycol/electrolyte solutions (PEG-ELS, GoLYTELY® or SF-ELS, NuLYTELY®) is a frequently prescribed preparation. This preparation, consisting of four liters of solution, is generally uncomfortable for the patient to ingest. They often complain of a sense of fullness, nausea, cramping, and vomiting, sometimes of such magnitude that they do not complete the prescribed regimen. Although these formulations are generally effective, they are not well tolerated by the patients. Failure to complete the regimen is a frequently named cause of inadequate bowel cleansing which often results in an inadequately cleansed colon for colonoscopy.

In an attempt to avoid the problems associated with the high volume types of preparations, other investigators have utilized ingestible preparations that consist of aqueous solutions of concentrated phosphate salts. The aqueous phosphate salt concentrate produces a tremendous osmotic effect on the intra-luminal contents of the bowel and therefore, evacuation of the bowel occurs with a large influx of water and electrolytes into the colon from the body. These phosphate salt preparations have been developed for the purpose of decreasing the volume required in colonic purgations. One such preparation is comprised of 480 grams per liter monobasic sodium phosphate and 180 grams per liter dibasic sodium phosphate in stabilized buffered aqueous solution and is sold under the brand name Fleets Phospho-Soda®. Patients are typically required to take two (2) three ounce doses of this preparation, separated by a three to 12 hour interval for a total of six ounces (180 ml), which is a significant reduction compared to the large one gallon volumes required by the high volume preparations. Additionally, non-aqueous tablet or capsule formulations of sodium phosphates and sulfates have been used (*see e.g.,* U.S. Patent Nos. 5,997,906, 6,162,464, and 5,616,346).

Another drawback of these prior art preparations is their unpleasant, bitter, saline taste. This can promote nausea and vomiting in sensitive patients-thereby preventing ingestion. It is difficult to overcome this unpleasant taste, even the most common natural sweeteners such as glucose, fructose, saccharose, and sorbitol could change the osmolarity of these orally administered solutions resulting in potentially dangerous electrolyte imbalances. These small volume phosphate solutions and non-aqueous formulations have been shown to cause massive electrolyte and fluid shifts that are clinically significant to the patient. These may result in permanent, severe kidney damage and even death (*see e.g.,* Markowitz et al., 2005, J. Am. Soc. Nephro/. 16:3389-3396; Ahmed et a/., 1996, Am. J. Gastroenterol., 91:1261-1262; Boivin et al., 1998, Am. J. Gastroenterol., 93:2577-2579; and Orias et al., 1999, Am. J. Nephrol. 19:60-63)*.*

These solutions are hyperosmotic in that the electrolyte concentration of the solution is much higher than the electrolyte concentration in the human body. Commercially available products, such as Fleet's Phospho-Soda, and the solid dosage form such as Visicol tablets (sodium phosphate salts), are examples of small volume electrolyte preparations. Unfortunately, all of these products have been shown to cause clinically significant electrolyte disturbances and fluid shifts, and disturbances in cardiac and renal function when administered to patients.

A significant diarrhea volume is believed to be necessary to produce adequate cleansing of the colon. In order to produce profuse, diarrhea, large volume lavages, such as PEG based lavages, have been employed. However, since most patients are not comfortable with experiencing such diarrhea, researchers have attempted, over the past 20 years, to reduce the volume of PEG based lavage preparations by combining them with laxatives, most notably bisacodyl. In many of these initial attempts, the volume of the solution was maintained at four liters, even with the addition of bisacodyl. In other experiments, attempts were made to use smaller volumes of a PEG based solution without bisacodyl or an additional laxative. Generally, these attempts produced improved patient symptoms but reduced the quality of the colonoscopy below acceptable standards. For example, Vilien et al., (1999, Endoscopy, 22:168-170) published a study of 50 patients that compared a reduced volume GoLYTELY® regimen with their standard GoLYTELY® preparation for colonoscopy. Colonoscopists who were unaware of the cleansing regimen that the patients had received rated the cleansing efficacy. On the day before examination, all patients were given 10 mg bisacodyl followed by a liquid diet. Then, on the morning of the exam, patients drank either 1.5 or 3 liters of GoLYTELY® (depending upon the randomization schedule). The authors concluded that there was less complete cleansing when the lower lavage volume was used. However, it is not clear how well these two treatments cleansed the bowel in comparison to the standard four liters of lavage solution alone.

Others have combined colon-cleansing modalities to achieve a clean, well-tolerated, preparation. Adams et al., 1994 (Dis. Colon Rectum, 37:229-234) compared colon preparation with bisacodyl followed eight hours later by two liters of GoLYTELY® to the "standard" four liters of GoLYTELY®. These researchers found that when patients received the bisacodyl 28-30 hours before examination and were placed on a clear liquid diet for more than 30 hours before examination, the quality of bowel cleansing between the two preparations appeared to be equivalent. The bisacodyl plus two liters GoLYTELY® method was better tolerated by patients. However, patients who received the bisacodyl plus two liters of GoLYTELY® but were not restricted to liquids for more than 30 hours before examination, did not have satisfactory preparation. In a similar study the results of colon cleansing were judged to be of the same visual quality, but the patients did not find any improvement in their level of discomfort (Gründel, et al., 1997, Dis. Colon Rectum, 40:1348-52).

Other studies have failed to find an adequate and effective combination of physician and patient assessments when a laxative is used in conjunction with a reduced lavage volume. Indeed, Bokemeyer, et al., 2000 (Verdauungskrankheiten, 18:17-24) found that the laxative plus reduced lavage volume resulted in a colonoscopy preparation with a smaller volume of PEG-lavage solution in combination with a laxative (X-Prep) that produced significantly worse results. (*See also* Lind, et al., 1990, Tidsskr Nor Laegeforen, 110:1357-1358; Brady, et al., 1987, Ann. Clin. Research. 19:34-38).

Thus, despite these attempts, improved patient symptoms have not necessarily follow the use of reduced volumes of lavage fluids with laxative pretreatment. Nor does the combination reliably produce a colon preparation that is as good as that achieved when a large volume lavage solution is used.

For virtual colonoscopy it is important that the bowels and colon be thoroughly cleansed of fecal material and that there be minimal or no residual liquid in the colon (*see,* Fenlon, et al., 1999, N. Engl. J. Med. 341:1496-1503; Lefeve, et al., 2006, Onkologic 29:281-286). In particular, it is essential that as much residual liquid and fecal matter as possible be removed from the colon to permit adequate visualization of the intestinal mucosa in order to obtain satisfactory radiographs of the colon by the method of virtual colonoscopy. Residual fluid in the colon can hinder the physician in observing polyps in the colon and therefore a procedure that results in very little remaining fluid in the colon is highly desirable. For example, Macari *et al.* (2001) and Gryspeerdt *et al.* (2002) report that the amount of residual fluid after a 4-liter volume prep in virtual colonoscopy is unacceptable as it hinders the radiological evaluation. Unlike optical colonoscopy, excess fluid cannot be removed in virtual colonoscopy. Macari, et al., 2001, Radiology, 218(1):274-7; Gryspeerdt, et al., 2002, JBR-BTR, 85:289-96.

Because patients have not optimally tolerated the methods used heretofore for cleansing their colon in preparation for virtual colonoscopy, and the methods described above leave too high a residual amount of fluid in the colon or have potentially dangerous side effects, there remains a need for methods of effectively cleansing a colon in preparation for virtual colonoscopy.

### SUMMARY OF THE INVENTION

It has been discovered that a specific regimen of administering a reduced volume of an osmotic lavage solution, followed by a bowel movement, and subsequently administering a stimulant laxative, produces a superior combination of an adequately cleansed colon with minimal colonic fluid remaining, and an improved patient acceptance of the procedure. This discovery has been exploited to develop the present invention, which relates to the preparation of the colon for examination by virtual colonoscopy, and to the visualization of the interior of an adequately prepared colon.

In one aspect of the invention, an osmotic laxative is orally administered to a mammal and is allowed to produce a bowel movement. Only after the bowel movement takes place is an effective amount of a stimulant laxative orally administered to the mammal. Afterwards, the mammal is allowed to evacuate the colon, whereby the colon is adequately cleansed to permit virtual colonoscopy. In one embodiment, the method comprises the further step of administering a clear liquid to the mammal after administration of the effective amount of the stimulant laxative.

The osmotic laxative is an aqueous suspension of a PEG that is a solid at room temperature and the effective amount of the osmotic laxative is from about 50 grams to about 400 grams of PEG in about two liters of an isotonic solution. In some embodiments, the osmotic laxative is administered in divided doses of PEG, each dose comprising from about 68 grams to about 75 grams of PEG in about 8 fluid ounces of isotonic solution. This dose is administered over about a two-hour period. In other embodiments, the osmotic laxative is PEG 3350. In one embodiment, the effective amount of osmotic laxative is about 119 g to about 221 g of PEG 3350 in about 2 liters of an aqueous solution.

The stimulant laxative is aloe, bisacodyl, casanthranol, cascara aromatic fluid extract, cascara sagrada bark, cascada sagrada extract, cascara sagrada fluid extract, castor oil, danthron, dehydrocholic acid, phenolphthalein, senna, sennosides A and B, or picosulfate. In certain embodiments, the stimulant laxative comprises bisacodyl. In particular embodiments the effective amount of bisacodyl is from about 5 mg to about 40 mg, or from about 10 mg to about 20 mg.

In other embodiments, a contrast agent is included in what is administered to the subject. In certain embodiments, the contrast agent is a tag for residual fecal matter. In other embodiments, the contrast agent is a tag for residual fluid. In a specific embodiment, the tag for residual fecal material is a barium agent. In another specific embodiment, the tag for residual fluid is a water-soluble, iodinated agent. In some embodiments, an anti-bubbling agent such as semithicone is included in what is administered to the subject. In some embodiments, the tags for residual fecal material and residual fluid and the anti-bubbling agent are used together or in any possible combinations.

In yet another aspect, the components used in cleansing the colon for virtual colonoscopy may be packaged as a kit comprising a concentrated solution of the osmotic laxative, a stimulant laxative, and at least one contrast agent. The kit may further contain semithicone in one embodiment. The osmotic laxative in the kit comprises PEG. In another embodiment, the stimulant laxative included in the kit comprises bisacodyl. In yet other embodiments, the kit further includes instructions for preparation for virtual colonoscopy.

In still another aspect, the invention provides a method of visualizing the interior of the colon of a mammal. This method comprises orally administering an effective amount of an osmotic laxative; allowing the laxative to produce a bowel movement; orally administering an effective amount of a stimulant laxative; allowing the mammal to evacuate its colon; and then performing virtual colonoscopy on the mammal. In some embodiments, conventional colonoscopy is performed after virtual colonoscopy to remove or examine any lesions or polyps detected.

### DETAILED DESCRIPTION

It has now been determined that safe and effective cleansing of the colon in preparation for virtual colonoscopy without large amounts of residual fluid can occur without the ingestion of large volumes of lavage solutions, without the unpleasant, bitter, and dangerous hypertonic salt solutions. It has been determined that a specific regimen of administering a reduced volume of an osmotic lavage solution, followed by a bowel movement, and subsequently administering a stimulant laxative, produces a superior combination of an adequately cleansed colon with minimal colonic fluid remaining, and an improved patient acceptance of the procedure. Furthermore, the degree of colonic cleansing is also adequate for optical or conventional colonoscopy on the same day, after the virtual colonoscopy.

This discovery has been exploited to develop the present invention, which relates to the preparation of the colon for examination by virtual colonoscopy. This regimen of: (1) lavage; (2) a biologically defined interval; and (3) a stimulant laxative produces a dryer and cleaner colon that is better for virtual colonoscopy than do prior art procedures. Additionally, the present regimen results in reduced occurrence of most common negative symptoms experienced by patients. This dual modality treatment is most advantageous if the timing of the administration of its two components is properly controlled by waiting for a bowel movement. This finding is highly unexpected and had not been previously appreciated in the art of virtual colonoscopy. While reference is typically made here in to "colon cleansing" it is understood that this invention will have value in cleansing the entire intestinal tract and rectum, from the duodenum to the anus, inclusive.

As described above, an initial step of the regimen is administration of an osmotic laxative. Although not being bound by any particular theory, osmotic laxatives are understood to work by reducing the amount of water absorbed from the bowel and so increasing the amount of water in the stools. This makes them softer and easier to pass. For examples some osmotic laxatives include indigestible carbohydrates, such as lactulose, and sorbitol, and saline laxatives based on sodium phosphate, magnesium citrate, or other magnesium salts, glycerol, and polyether polyols. These laxatives are commercially available, *e.g*., from Apotex, Weston F1, and Cumberland Pharmaceuticals Inc., Nashville, TN.

One useful group of osmotic laxatives is the polyether polyols. Polyether polyols are compounds formed through the polymerization of ethylene oxide, propylene oxide, butylene oxide, or other cyclic ethers, separately or as mixtures, with compounds having one or more reactive hydrogens (*i.e.,* a hydrogen atom bonded to nitrogen, oxygen, phosphorus, sulfur, etc.) to form polyethers (*i.e*., compounds with two or more ether bonds). These polyether polyols contain more than one hydroxyl radical after polymerization. Polyether polyols having molecular weight ranges of about 900 to about 20,000 are useful in the invention. Examples of polyether polyols are polyethylene glycol, polypropylene glycol, polyethylene-polypropylene glycol block co-polymers and random polymers, and polybutylene polyols.

The polyether polyol used in the invention, is polyethylene glycol ("PEG"). Any food- or pharmaceutical-grade PEG polymer may be employed in the compositions contemplated herein. Polymers of relatively high molecular weight (*e.g*., above about 900) that are solid at room temperature and soluble in (or miscible with) water at room temperature are useful in the described regimen. Polymers having an average molecular weight of at least 1000 (and generally no greater than 20,000) can be used. In particular, PEGs with molecular weights in the range of from about 3000 to about 8000 may be used. PEG 3350 (the numeric designation identifying the average molecular weight) has been shown to be useful.

Another example of polyether polyol is polyethylenepolypropylene glycol ("PPG"). PPG is also known under the name "pluronic," "pluronic F68," or "poloxamer 188". Pluronic F68 has a molecular weight of 8350, and is used as a food additive due to its surfactant, lubricating and non-foaming properties. Pluronic F68 can be purchased from various sources (*e.g.,* BASF, Mount Olive, New Jersey).

For the regimen described herein to be effective in producing a properly cleansed and dry bowel for virtual colonoscopy, following osmotic laxative administration, a bowel movement must occur before administration of the stimulant laxative. A bowel movement typically occurs within 24 hours. Sometimes the bowel movement occurs within 18 hours, at other times, within 12 hours, within six hours, or less. In the case of the polyethylene glycol-based lavages (GoLYTELY® and NuLYTELY®), the bowel movement usually occurs rapidly, within one half to three hours. Without waiting for the bowel to empty itself after administration of the osmotic laxative before administering the stimulant laxative results in colons that are not well cleansed (Brady, et al., 1987, Ann. Clin. Research, 19:34-38).

After the bowel movement has occurred the stimulant laxative is administered. Without being bound to any particular theory, stimulant laxatives are understood to cause rhythmic muscle contractions in the large intestines. Effective doses of some stimulant laxatives include: about 250 mg to about 1000 mg of aloe; about 5 mg to about 80 mg of bisacodyl; about 30 mg to about 360 mg of casanthranol; about 2 ml to about 24 ml of cascara aromatic fluid extract; about 300 mg to about 4000 mg of cascara sagrada bark; about 300 mg to about 2000 mg of cascada sagrada extract; about 0.5 ml to about 5 ml of cascara sagrada fluid extract; about 15 ml to about 240 ml of castor oil; about 75 mg to about 300 mg of danthron; about 250 mg to about 2000 mg of dehydrocholic acid; about 30 mg to about 1000 mg of phenolphthalein; about 50 mg to about 400 mg of sennosides A and B; about 0.6 g to about 5 g of Senna; and about 1 mg to about 100 mg of picosulfate. These stimulant laxatives are commercially available from, *e.g*., Boehinger Engleheim, Ingleheim, Germany; Purdue Pharma, Stamford, Connecticut; Schering Plough, Kenilworth, New Jersey. Of course, larger or smaller doses may be used, as necessary, to produce a bowel movement while avoiding unnecessary discomfort.

One such useful stimulant laxative is bisacodyl, which is commercially available without prescription. Bisacodyl is available in tablets, suppositories, and in premixed enema formulations. Bisacodyl enemas are usually effective to produce a bowel movement in about 20 minutes; suppositories usually produce a bowel movement in about an hour, and oral administration of a tablet usually results in a bowel movement in about three to six hours. Bisacodyl stimulates the intestines and rectum to produce a bowel movement. Stimulant laxatives, alone, can be effective to treat constipation, but have not been effective to cleanse satisfactorily a patient's colon prior to colonoscopic examination or surgical procedure.

Oral administration of about 5 mg to about 40 mg of bisacodyl is usually effective to produce a bowel movement within about three to about six hours after administration. However, about 5 mg to about 80 mg or about 10 mg to about 20 mg of bisacodyl may be administered to a patient to produce a bowel movement.

Gas bubbles that are present in the residual fluid may be reduced because the attenuation of the X-rays is different between fluid and gas bubbles, making it difficult for a radiologist to clearly demarcate the fluid if it is full of bubbles. Addition of simethicone reduces unwanted gas bubbles. Simethicone is the active ingredient in several over the counter gas-reducing products (*i.e*., Phazyme®; Flatulex®; Mylicon®; Gas-X®; Mylanta Gas®) A dose of about 20 mg to about 100 mg can be taken orally by the subject in a tablet or even chewing gum. Simethicone can be included in the composition of the present invention so as to provide a dose of about 20 mg to about 100 mg. The simethicone can be directly added to the prep at any step. For example, it can be administered when the osmotic and/or stimulant laxative(s) is administered, or it can be added before or after the administration of the osmotic and/or stimulant laxative(s). It can also be administered at more than one step of the method, or at each step of the method.

In order to identify residual fecal matter and/or residual fluid and not confuse them with an actual polyp, a "tag" can be included in the prep for identifying residual fecal materials or residual fluids. Such tags alter the contrast of these residues, so that they can be more readily discriminated from normal or diseased tissues by the radiologist. For example, a fecal tag (barium) and/or a residual fluid tag (*e.g*., Gastrografin®) can be consumed as part of the bowel prep to enhance the radiologist's ability to discriminate lesions from feces and fluids, using the virtual colonoscopy imaging software. The subjects ingest about 250 ml of a 2% CT barium solution (for example, SCAN C® Preservative-Free Barium Sulfate Suspension, Lafayette Pharmaceuticals, Lafayette IN) to enhance the sensitivity and specificity of the virtual colonoscopy technique to detect polyps. (Pickhardt et al., 2003, N. Engl. J. Med., 349:2191). The tag can be directly added to the prep at any step. For example, it can be administered when the osmotic and/or stimulant laxative(s) is administered, or it can be added before or after the administration of the osmotic and/or stimulant laxative(s).

Subjects can also ingest a water-soluble, iodinated contrast medium comprising Diatrizoate (for example, Gastrografin® Diatrizoate Meglumine & Diatrizoate Sodium Solution USP, Bracco Diagnostics, Princeton, NJ) to enhance the sensitivity and specificity of the virtual colonoscopy method to detect polyps. The diatrizoate solution alters the radiographic contrast of retained fluid, making it easier to discriminate fluid from polyps (Pickhardt *et al.,* 2003, *ibid.*)*.* The water-soluble, iodinated contrast medium can be directly added to the prep at any step. For example, it can be administered when the osmotic and/or stimulant laxative(s) is administered, or it can be added before or after the administration of the osmotic and/or stimulant laxative(s).

Since the virtual colonoscopy prep produces a clean colon, the virtual colonoscopy examination may begin any time after the patient's diarrheal effluent is clear of solid debris. This is usually accomplished in about two hours after completion of the prep. Once the colon is cleaned, the virtual colonoscopy exam can occur any time thereafter. For practical reasons and patient comfort, the exam should occur within 36 hours of initiating the prep. Longer periods are allowed, if necessary, if the patient is allowed to drink amounts of rehydrating solutions that also contain an energy source, such as glucose. In this manner the effects on the patient of prolonged fasting can be overcome.

Virtual colonoscopy examinations are performed using state of the art technology. Briefly, the CT scanner used is at minimum a four- or eight-channel CT scanner. For example, a multi-row detector scanner (such as a Marconi MX 8000 (Philips Medical Systems, N.A., Bothell, WA) or the Light-Speed and LightSpeed Ultra (General Electric Medical Systems Milwaukee, WI) can be used. A delivery system enema tip (with lubricant for ease of insertion) is used for insufflating gas (CO₂ or room air) into the colon. The tip of the catheter is inserted into the rectum and then the patient is placed in the supine position. For example, if an EZ-EM Protocol Colon Insufflator ® (E-Z-EM Inc., 1111 Marcus Avenue, Suite LL26, Lake Success, NY 11042) or similar automatic device is used to expand the colon for CT images using CO₂, the pressure is slowly increased to 20 mmHg to 25 mmHg. Patient comfort dictates the speed at which insufflation takes place to an optimum volume of 2 to 4 liters. Alternatively, the patient or a technician can use a hand bulb to insufflate the colon with room air or some other appropriate gas before each scan. When insufflation is complete, a scout image is obtained to assess the colon for proper insufflation. Additional insufflation and a new scout image may be needed when colonic distention is considered insufficient. When the degree of distention is deemed adequate by the physician or technician, the virtual colonoscopy images are acquired, first in the supine and then in the prone position.

Each data set (supine and prone) can be acquired in a single breath-hold period. Total time spent by the patient in the CT suite is about 45 minutes, on average.

The following are examples of specific parameters used for CT scanning with the Marconi MX8000 system.

**TABLE 1**

| | |
|---|---|
| Collimation: | 1.5-2 mm |
| Pitch Factor: | 6:1 (4 detector) |
| mAs: | 50-80 |
| kVp: | 120 |
| Matrix: | 512 x 512 |
| Reconstruction interval: | 1 mm or less |

The virtual colonoscopy examinations are interpreted by a trained radiologist or technician, using dedicated workstations specifically designed for this purpose. The workstations allow simultaneous visualization of the axial images acquired directly from the scanner (raw data), as well as two-dimensional (2D) and three-dimensional (3D) renderings that permit "fly throughs" of the colon and are necessary for distinguishing complex folds from true lesions, such as polyps. Image processing and interpretation are performed on one of the commercially available CT colonography systems such as a Viatronix V3D, version 1.3 (Viatronix, Stony Brook, NY). If the colonoscopist chooses to prep the patients with contrast media or other compounds that "tag" stool or water, a software package that achieves electronic subtraction of these interferences can be used. Newer software systems that possess "artificial intelligence" and that can detect polyps and other pathologies and then alert the colonoscopists to their presence can also be used.

The colonoscopist can record all of the detected lesions, their location (for example, in the cecum, ascending, transverse, descending, sigmoid and rectum), as well as the lesion's size, using electronic calipers and measurement tools available in the workstations. A conventional colonoscopy can be carried out after virtual colonoscopy to treat the lesions.

The following examples illustrate modes of practicing the present invention.

### EXAMPLES

### Example 1

Consecutive patients scheduled for virtual colonoscopy were recruited into a clinical study (F38 VC-002). Patients consumed a light breakfast the day before virtual colonoscopy, then started drinking 2 liters of NuLYTELY® (polyethylene glycol 3350, NF, 210 g, sodium chloride, USP 5.60 g, sodium bicarbonate, USP 2.86 g, potassium chloride, USP 0.74 g, and optionally, 1 g of a flavor ingredient; Braintree Labs, Inc., Braintree, MA) between noon and 2 PM after a clear liquid lunch. They began consuming one glassful (8 oz) of the NuLYTELY® solution every 10 to 15 minutes until they consumed a total of 2 liters of the solution. Most of the patients reported that they had one to several bowel movements during or after consuming the solution. Study patients took the four enteric-coated bisacodyl tablets about three hours after completing the NuLYTELY®. The patients were instructed to consume nothing by mouth, except clear liquids, thereafter.

Virtual colonoscopy examinations were performed with a multi-row detector scanner (Marconi MX 8000, Philips Medical Systems, N.A., Bothell, WA) with colonic CO₂ insufflation at about 20 mmHg to about 25 mmHg. After a scout image, each data set (supine and prone) was acquired in a single breath-hold period. The virtual colonoscopy examinations were interpreted using workstations that allowed simultaneous visualization of the axial images acquired directly from the scanner (raw data), as well as 2D and 3D renderings. The amount of residual stool and fluid in five segments of the colon were quantified using a five-point scale (Gryspeerdt *et al.,* 2002, *ibid.*).

Seven patients completed this regimen. The ratings of the residual stool and residual fluid remaining in the colon after the clean out were determined by the radiologist and are presented in the following table. These results are compared to those of Gryspeerdt *et al.* (*ibid*.) who employed a 4-liter preparation containing PEG and electrolytes in 20 patients.

**TABLE 2**

| Effects of Different Bowel Treatments on Residual Stool and Fluid in Virtual Colonoscopy | | |
|---|---|---|
| Bowel Treatment | % Patients with absent or small stool residue | % Patients with absent or small fluid residue |
| F38 VC-002: 2 L NuLYTELY® followed by bisacodyl | 86% | 86% |
| Gryspeerdt: 4 L PEG Electrolyte | 80% | 0% |

As Table 2 shows, the 2 liters NuLYTELY® plus bisacodyl bowel treatment was an improvement over the 4 liters of PEG plus electrolytes treatment with regard to its effectiveness to produce a colon free of stool. However, when the amounts of residual fluid are compared, the 2 liters NuLYTELY® plus bisacodyl bowel treatment was superior to a 4 liter NuLYTELY® treatment. None of the patients using the method of the invention had excess fluid in their colons. In fact, all but one patient of the seven had little or no residual fluid. The remaining patient had a "moderate" amount of fluid localized only in one colonic segment. The investigators rated all of these patients as having good to excellent preparations. No patient required repeated preparation.

Conventional colonoscopy results were similar. All conventional colonoscopy exams were considered complete, and most were considered good or excellent. The amount of stool present was judged absent or small in most of the segments. Only one had an excess of stool, which was removed by aspiration. No fluid or only small amounts were detected in 99% of the segments.

Thus, same day virtual colonoscopy and conventional colonoscopy offers physicians a way to screen, identify and, if necessary, remove colonic polyps with a single bowel cleansing preparation, improving patient convenience.

### Example 2

In this study, the methods of the invention is compared with another method of preparing the bowel for virtual colonoscopy.

All patients consume a light breakfast and a clear liquid lunch the day before virtual colonoscopy. All patients are then administered their treatments.

In the first treatment group (Group 1), study patients consume two liters of NuLYTELY® (polyethylene glycol 3350, NF, 210 g, sodium chloride, USP 5.60 g, sodium bicarbonate, USP 2.86 g, potassium chloride, USP 0.74 g, and optionally, 1 g of a flavor ingredient; Braintree Labs, Inc., Braintree, MA). Group 1 patients are instructed to wait for a bowel movement to occur after taking the 2 liters of NuLYTELY®. Most patients have at least one bowel movement while drinking the NuLYTELY®. After the patients complete drinking the NuLYTELY® and have had at least one bowel movement, they then take 20 mg of bisacodyl.

In Group 2, the patients are administered the standard dose of NuLYTELY® for bowel cleansing (4 liters). No stimulant laxative is administered after the ingestion of NuLYTELY®.

For all patients, the virtual colonoscopies are generally scheduled on the morning of the next day. Virtual colonoscopy examinations are performed with a multi-row detector scanner with colonic CO₂ insufflation at about 20 mmHg to about 25 mmHg. After a scout image, each data set (supine and prone) is acquired in a single breath-hold period. The virtual colonoscopy examinations are interpreted using workstations that allow simultaneous visualization of the axial images acquired directly from the scanner (raw data), as well as 2D and 3D renderings. The amount of residual stool and fluid in five segments of the colon are quantified using a five-point scale (Gryspeerdt *et al.,* 2002, *ibid.*).

Results similar to Table 2 are expected where Group 1 patients have equivalent or superior cleansing compared to Group 2 with much less fluid in the colon, allowing for a superior virtual colonoscopy examination. A further advantage of the method is that the total time of patient preparation is much reduced.

## Claims

1. Use of an osmotic laxative and a stimulant laxative for preparing the colon of a mammal for virtual colonoscopy
a) wherein an effective amount of an osmotic laxative is administered to the mammal;
b) wherein the osmotic laxative is allowed to produce a bowel movement;
c) wherein an effective amount of a stimulant laxative is orally administered to the mammal; and
d) wherein the mammal is allowed to evacuate the colon,
whereby there is minimal residual colonic fluid present; and whereby the colon is adequately cleansed to permit virtual colonoscopy,
wherein the effective amount of the osmotic laxative is from 50 grams to 400 grams of a polyethylene glycol that is solid at room temperature in two liters of an isotonic solution, and
wherein the stimulant laxative is selected from the group consisting of aloe, bisacodyl, casanthranol, cascara aromatic fluid extract, cascara sagrada bark, cascada sagrada extract, cascara sagrada fluid extract, castor oil, danthron, dehydro- cholic acid, phenolphthalein, senna, sennosides A and B, and picosulfate.

2. The use of claim 1, wherein a clear liquid is administered to the mammal after step c.

3. The use according to claim 1, wherein the PEG is administered in divided doses, each dose comprising from 68 grams to 75 grams of PEG in 236.6 ml of isotonic solution over a two hour period.

4. The use of claim 1, wherein the stimulant laxative is bisacodyl, preferably wherein the effective amount of bisacodyl is from 5 mg to 40 mg.

5. The use of claim 1, wherein the osmotic laxative is PEG 3350 and the effective amount of osmotic laxative is 2 litres of an aqueous solution comprising 119 grams to 221 grams of PEG 3350; the stimulant laxative is bisacodyl, and the effective amount of bisacodyl is from 10 mg to 20 mg.

6. The use of claim 1, further comprising the administration of simethicone.

7. The use of claim 1, further comprising the administration of a contrast agent.

8. The use of claim 7, wherein the contrast agent is a tag for residual fecal matter, preferably comprising a barium contrast agent.

9. The use of claim 7, wherein the contrast agent is a tag for residual fluid, preferably comprising a water-soluble, iodinated contrast agent.

10. The use of claim 9, wherein the water-soluble iodinated contrast agent comprises diatrizoate.

11. A kit comprising an osmotic laxative, a stimulant laxative, and at least one contrast agent, for use in cleansing a colon in preparation for virtual colonoscopy,
wherein the amount of the osmotic laxative is from 50 grams to 400 grams of a polyethylene glycol that is solid at room temperature in two liters of an isotonic solution, and
wherein the stimulant laxative is selected from the group consisting of aloe, bisacodyl, casanthranol, cascara aromatic fluid extract, cascara sagrada bark, cascada sagrada extract, cascara sagrada fluid extract, castor oil, danthron, dehydro- cholic acid, phenolphthalein, senna, sennosides A and B, and picosulfate.

12. The kit of claim 11, wherein the osmotic laxative is PEG and wherein the stimulant laxative is bisacodyl.

13. Use of an osmotic laxative and a stimulant laxative for preparing the colon of a mammal for virtual colonoscopy
a) wherein an effective amount of an osmotic laxative is administered to the mammal;
b) wherein the osmotic laxative is allowed to produce a bowel movement;
c) wherein an effective amount of a stimulant laxative is orally administered to the mammal; and
d) wherein the mammal is allowed to evacuate the colon resulting in minimal residual colonic fluid present and the colon is adequately cleansed to permit virtual colonoscopy; and
e) wherein the interior of the colon of the mammal is visualized by virtual colonscopy,
wherein the effective amount of the osmotic laxative is from 50 grams to 400 grams of a polyethylene glycol that is solid at room temperature in two liters of an isotonic solution, and
wherein the stimulant laxative is selected from the group consisting of aloe, bisacodyl, casanthranol, cascara aromatic fluid extract, cascara sagrada bark, cascada sagrada extract, cascara sagrada fluid extract, castor oil, danthron, dehydro- cholic acid, phenolphthalein, senna, sennosides A and B, and picosulfate.

## Patentansprüche

1. Die Verwendung eines osmotischen Abführmittels und eines stimulierenden Abführmittels zur Vorbereitung des Dickdarms eines Säugetiers auf eine virtuelle Koloskopie,
a) worin dem Säugetier eine wirksame Menge eines osmotischen Abführmittels verabreicht wird;
b) worin dem osmotischen Abführmittel erlaubt wird, eine Darmbewegung zu erzeugen;
c) worin dem Säugetier eine wirksame Menge eines stimulierenden Abführmittels oral verabreicht wird; und
d) worin dem Säugetier erlaubt wird, seinen Dickdarm zu entleeren,
wobei die restliche vorliegende Dickdarmflüssigkeit minimal ist; und wobei der Dickdarm ausreichend gereinigt ist, um virtuelle Koloskopie zu gestatten,
worin die wirksame Menge des osmotischen Abführmittels zwischen 50 Gramm und 400 Gramm eines bei Raumtemperatur festen Polyethylenglykols in zwei Litern einer isotonischen Lösung liegt und
worin das stimulierende Abführmittel ausgewählt wird aus der Gruppe bestehend aus Aloe, Bisacodyl, Casanthranol, aromatischem flüssigem Cascara-Extrakt, Cascara-sagrada-Rinde, Cascara-sagrada-Extrakt, flüssigem Cascara-sagrada-Extrakt, Rizinusöl, Danthron, Dehydrocholsäure, Phenolphthalein, Sennes, Sennoside A und B und Picosulfat.

2. Die Verwendung aus Anspruch 1, worin dem Säugetier nach Schritt c eine klare Flüssigkeit verabreicht wird.

3. Die Verwendung gemäß Anspruch 1, worin das PEG in getrennten Dosen verabreicht wird, jede Dosis umfassend zwischen 68 Gramm bis 75 Gramm PEG in 236,6 ml isotonischer Flüssigkeit über eine zweistündige Periode.

4. Die Verwendung aus Anspruch 1, worin das stimulierende Abführmittel Bisacodyl ist, vorzugsweise worin die wirksame Menge von Bisacodyl zwischen 5 mg und 40 mg liegt.

5. Die Verwendung aus Anspruch 1, worin das osmotische Abführmittel PEG 3350 ist und die wirksame Menge von osmotischem Abführmittel 2 Liter einer wässrigen Lösung ist umfassend 119 Gramm bis 221 Gramm PEG 3350; das stimulierende Abführmittel Bisacodyl ist und die wirksame Menge von Bisacodyl zwischen 10 mg und 20 mg liegt.

6. Die Verwendung aus Anspruch 1, weiter umfassend die Verabreichung von Simethicon.

7. Die Verwendung aus Anspruch 1, weiter umfassend die Verabreichung eines Kontrastmittels.

8. Die Verwendung aus Anspruch 7, worin das Kontrastmittel ein Marker für restliche Fäkalmaterie ist, vorzugsweise umfassend ein Barium-Kontrastmittel.

9. Die Verwendung aus Anspruch 7, worin das Kontrastmittel ein Marker für restliche Flüssigkeit ist, vorzugsweise umfassend ein wasserlösliches jodiertes Kontrastmittel.

10. Die Verwendung aus Anspruch 9, worin das wasserlösliche jodierte Kontrastmittel Diatrizoat enthält.

11. Ein Kit umfassend ein osmotisches Abführmittel, ein stimulierendes Abführmittel und mindestens ein Kontrastmittel, zur Verwendung in der Reinigung des Dickdarms zur Vorbereitung auf eine virtuelle Koloskopie,
worin die Menge des osmotischen Abführmittels zwischen 50 Gramm und 400 Gramm eines bei Raumtemperatur festen Polyethylenglykols in zwei Litern einer isotonischen Lösung liegt und
worin das stimulierende Abführmittel ausgewählt ist aus der Gruppe bestehend aus Aloe, Bisacodyl, Casanthranol, aromatischem flüssigem Cascara-Extrakt, Cascara-sagrada-Rinde, Cascara-sagrada-Extrakt, flüssigem Cascara-sagrada-Extrakt, Rizinusöl, Danthron, Dehydrocholsäure, Phenolphthalein, Sennes, Sennoside A und B und Picosulfat.

12. Das Kit aus Anspruch 11, worin das osmotische Abführmittel PEG ist und worin das stimulierende Abführmittel Bisacodyl ist.

13. Die Verwendung eines osmotischen Abführmittels und eines stimulierenden Abführmittels zur Vorbereitung des Dickdarms eines Säugetiers auf eine virtuelle Koloskopie,
a) worin dem Säugetier eine wirksame Menge eines osmotischen Abführmittels verabreicht wird;
b) worin dem osmotischen Abführmittel erlaubt wird, eine Darmbewegung zu erzeugen;
c) worin dem Säugetier eine wirksame Menge eines stimulierenden Abführmittels oral verabreicht wird; und
d) worin dem Säugetier erlaubt wird, seinen Dickdarm zu entleeren, wobei die restliche vorliegende Dickdarmflüssigkeit minimal ist und der Dickdarm ausreichend gereinigt ist, um virtuelle Koloskopie zu gestatten; und
e) worin das Innere des Dickdarms des Säugetiers mittels virtueller Koloskopie visualisiert wird,
worin die Menge des osmotischen Abführmittels zwischen 50 Gramm und 400 Gramm eines bei Raumtemperatur festen Polyethylenglykols in zwei Litern einer isotonischen Lösung liegt und
worin das stimulierende Abführmittel ausgewählt wird aus der Gruppe bestehend aus Aloe, Bisacodyl, Casanthranol, aromatischem flüssigem Cascara-Extrakt, Cascara-sagrada-Rinde, Cascara-sagrada-Extrakt, flüssigem Cascara-sagrada-Extrakt, Rizinusöl, Danthron, Dehydrocholsäure, Phenolphthalein, Sennes, Sennoside A und B und Picosulfat.

## Revendications

1. Utilisation d'un laxatif osmotique et d'un laxatif stimulant pour la préparation du côlon d'un mammifère pour une coloscopie virtuelle
a) dans laquelle une quantité efficace d'un laxatif osmotique est administrée au mammifère ;
b) dans laquelle on laisse le laxatif osmotique produire une défécation ;
c) dans laquelle une quantité efficace d'un laxatif stimulant est administrée par voie orale au mammifère ; et
d) dans laquelle on laisse le mammifère vider son côlon,
moyennant quoi la quantité de fluide colique résiduel présent est minimale ; et moyennant quoi le côlon est nettoyé de manière appropriée pour permettre une coloscopie virtuelle,
dans laquelle la quantité efficace du laxatif osmotique va de 50 grammes à 400 grammes d'un polyéthylène glycol qui est solide à température ambiante dans deux litres d'une solution isotonique, et
dans laquelle le laxatif stimulant est choisi dans le groupe comprenant l'aloès, le bisacodyl, le casanthranol, un extrait fluide aromatique de cascara, l'écorce de cascara sagrada, un extrait de cascara sagrada, un extrait fluide de cascara sagrada, l'huile de ricin, la dantrone, l'acide dehydrocholique, la phénolphtaléine, le séné, les sennosides A et B, et le picosulfate.

2. Utilisation selon la revendication 1, dans laquelle un liquide clair est administré au mammifère après l'étape c.

3. Utilisation selon la revendication 1, dans laquelle le polyéthylène glycol est administré en doses divisées, chaque dose comprenant de 68 grammes à 75 grammes de polyéthylène glycol dans 236,6 ml d'une solution isotonique sur une période de deux heures.

4. Utilisation selon la revendication 1, dans laquelle le laxatif stimulant est le bisacodyl, de préférence dans laquelle la quantité efficace de bisacodyl va de 5 mg à 40 mg.

5. Utilisation selon la revendication 1, dans laquelle le laxatif osmotique est le polyéthylène glycol 3350 et la quantité efficace de laxatif osmotique est de 2 litres d'une solution aqueuse comprenant de 119 grammes à 221 grammes de polyéthylène glycol 3350 ; le laxatif stimulant est le bisacodyl, et la quantité efficace de bisacodyl va de 10 mg à 20 mg.

6. Utilisation selon la revendication 1, comprenant en outre l'administration de siméthicone.

7. Utilisation selon la revendication 1, comprenant en outre l'administration d'un produit de contraste.

8. Utilisation selon la revendication 7, dans laquelle le produit de contraste est un marqueur pour la matière fécale résiduelle, et comprend de préférence un produit de contraste à base de baryum.

9. Utilisation selon la revendication 7, dans laquelle le produit de contraste est un marqueur pour un fluide résiduel, et comprend de préférence un produit de contraste iodé hydrosoluble.

10. Utilisation selon la revendication 9, dans laquelle le produit de contraste iodé hydrosoluble comprend du diatrizoate.

11. Kit comprenant un laxatif osmotique, un laxatif stimulant, et au moins un produit de contraste, destiné à une utilisation pour nettoyer un côlon pour la préparation à une coloscopie virtuelle,
dans lequel la quantité du laxatif osmotique va de 50 grammes à 400 grammes d'un polyéthylène glycol qui est solide à température ambiante dans deux litres d'une solution isotonique, et
dans lequel le laxatif stimulant est choisi dans le groupe comprenant l'aloès, le bisacodyl, le casanthranol, un extrait fluide aromatique de cascara, l'écorce de cascara sagrada, un extrait de cascara sagrada, un extrait fluide de cascara sagrada, l'huile de ricin, la dantrone, l'acide dehydrocholique, la phénolphtaléine, le séné, les sennosides A et B, et le picosulfate.

12. Kit selon la revendication 11, dans lequel le laxatif osmotique est le polyéthylène glycol et dans lequel le laxatif stimulant est le bisacodyl.

13. Utilisation d'un laxatif osmotique et d'un laxatif stimulant pour la préparation du côlon d'un mammifère à une coloscopie virtuelle
a) dans laquelle une quantité efficace d'un laxatif osmotique est administrée au mammifère ;
b) dans laquelle on laisse le laxatif osmotique produire une défécation ;
c) dans laquelle une quantité efficace d'un laxatif stimulant est administrée par voie orale au mammifère ; et
d) dans laquelle on laisse le mammifère vider son côlon, moyennant quoi la quantité de fluide colique résiduel présent est minimale et le côlon est nettoyé de manière appropriée pour permettre une coloscopie virtuelle ; et
e) dans laquelle l'intérieur du côlon du mammifère est visualisé par coloscopie virtuelle,
dans laquelle la quantité efficace du laxatif osmotique va de 50 grammes à 400 grammes d'un polyéthylène glycol qui est solide à température ambiante dans deux litres d'une solution isotonique, et
dans laquelle le laxatif stimulant est choisi dans le groupe comprenant l'aloès, le bisacodyl, le casanthranol, un extrait fluide aromatique de cascara, l'écorce de cascara sagrada, un extrait de cascara sagrada, un extrait fluide de cascara sagrada, l'huile de ricin, la dantrone, l'acide dehydrocholique, la phénolphtaléine, le séné, les sennosides A et B, et le picosulfate.
